# EUROPEAN PATENT SPECIFICATION

(11) **EP 3 764 975 B1**
(45) Date of publication and mention of the grant of the patent: **13.10.2021**
(21) Application number: 19704641.0
(22) Date of filing: 19.02.2019
(51) Int. Cl.: A61K 8/34, A61K 8/67, A61Q 17/00, A61K 31/455

(54) **A SANITIZER COMPOSITION**
DESINFEKTIONSZUSAMMENSETZUNG
COMPOSITION DE DÉSINFECTANT

(30) Priority: 13.03.2018 EP 18161444
(43) Date of publication of application: 20.01.2021
(73) Proprietor: Unilever Global IP Limited, Wirral, CH62 AZD (GB); Unilever IP Holdings B.V., 3013 AL Rotterdam (NL)
(72) Inventor: BAPAT, Mohini, Anand, Mumbai 400 099 Andheri East (IN); MAJUMDAR, Amitabha, Bangalore 560 066 Whitefield (IN); MATHAPATHI, Mruthyunjaya, Swamy, Bangalore 560 066 Whitefield (IN); RAUT, Janhavi, Sanjay, Bangalore 560 066 Whitefield (IN)
(74) Representative: Fijnvandraat, Arnoldus
(86) International application number: PCT/EP2019/054028
(87) International publication number: WO 2019/174867

(56) References cited:
- WO-A2-2012/152567
- US-A- 5 167 950
- US-A1- 2015 258 003

## Description

### Field of the invention

The invention relates to a sanitizer composition for topical use, i.e. for application onto the human skin, especially on hands for instantaneous disinfection of the body part while imparting long lasting immunity benefit.

### Background of the invention

Sanitizers, sometimes called disinfectants, antiseptics or antimicrobials are compositions that are applied to living tissues, e.g. skin, to reduce the possibility of infection. Sanitizers are generally used to denote compositions that provide instantaneous kill of germs on topical surfaces of animal or human body while disinfectants are generally used for the killing germs on non-living surfaces. Sanitizers often involve use of alcohol based microbiocides. Sanitizers are generally formulated with minimal amount of water or with no water. These are used by applying a few drops of the solution on to the topical surface e.g the hand and then rubbed together. During rubbing, the alcohol and other antimicrobial substances spread on the desired surface causing the antimicrobial action to take place. Parallelly, the alcohol evaporates leaving the surface disinfected without the need for rinsing the surface with water and subsequent drying as is necessary in the case of soap or other hand wash products. Sanitizers are useful in hospitals, clinics, nursing homes and other healthcare places where doctors, nurses and other healthcare workers need to disinfect their hands, often several times an hour, as they attend to one patient after another to ensure, minimizing the spread of disease through their contact. These are different from antibiotics by the latter's ability to be transported through the lymphatic system to destroy bacteria within the body.

Skin generally contains several different microorganisms in concentrations exceeding millions or even billions of colony forming units (cfu) per square centimetre (cm²). Many of these microorganisms are harmless, but there are also various pathogenic species present, such as E. *coli* or S. *aureus.* Also, other unwanted bacteria are found on the skin, such as S. *epidermidis,* which is generally non-pathogenic, but is thought to cause an unpleasant body odour.

Alcohols, usually ethanol or isopropanol are used in such compositions. Use of alcohol causes drying of the skin and can be unpleasant and irritating to some consumers. One of the skin care actives with a wide variety of beneficial uses is a vitamin B3 derivative e.g. niacinamide.

Vitamin B3 or the amide thereof (niacinamide) are included as a suitable ingredient for skin care products, because vitamin B3 and niacinamide are known to have several properties that are advantageous for the skin, such as the prevention of cellulite, enhancement of stratum corneum formation, treatment of skin irritation, and improvement of absorption of water into the skin. Niacinamide is also used as a skin lightening active. In addition, niacinamide is used in the treatment of many inflammatory skin conditions like Acne vulgaris, Psoriasis and Atopic dermatitis.

WO 2015/172801 A1 (Unilever) describes the use of niacinamide for inducing the secretion of anti-microbial peptides (AMPs) by keratinocytes when applied on an external surface of the human body.

US2015/258003 discloses a handwash composition that maintains or improves skin hydration, the composition comprising: from about 10 to about 98 percent by weight (wt. %) of at least one C₁₋₆ alcohol, based upon the total weight of the composition; at least one phytochemical with anti-inflammatory properties; at least one enzyme or coenzyme that aids in the formation of the stratum corneum; at least one deposition enhancer selected from the group consisting of polyolprepolymer-2, polyolprepolymer-14, and polyolprepolymer-15.

To counter the ill effects of alcohol in sanitizing compositions, it is advantageous to include a vitamin B3 compound or a derivative e.g. niacinamide therein. The present inventors found that it is difficult to incorporate a vitamin B3 compound or a derivative thereof in a high alcohol containing sanitizer. The difficulty is that when alcohol is included in higher than about 60 weight percent of the composition, vitamin B3 compound or its derivative tends to crystallise on storage and cause haziness of the composition. The present inventors through extensive experimentation found that the inclusion of specific non-ionic surfactants tends to alleviate the problem by possibly inhibiting the crystallisation of the vitamin compound.

WO2017202586 (Unilever) discloses a composition comprising (a) 20 to 90 wt. % of one or more C2 to C4 monohydric alcohols; (b) 0.5 to 10 wt. % vitamin B3 compound; (c) 1 to 20 wt. % of one or more polyols; and wherein the weight ratio of the vitamin B3 compound to the one or more polyols is in the range of 1:2 to 1:20 and wherein the viscosity of the composition is in the range of 0.5 Pa s (500 cps) to 18 Pa s (18000 cps) at 25°C wherein said monohydric alcohols are selected from ethanol, isopropyl alcohol and combinations thereof and further wherein the composition comprises a combination of ethanol and isopropyl alcohol.

While the above publication discloses inclusion of certain polyols to prevent the formation of deposits, the present invention is directed to minimization of haziness of the formulation during storage, and this has been solved in the present invention by inclusion of specific non ionic surfactants.

It is thus an object of the present invention to provide for a stable alcohol based sanitizer composition that has the beneficial properties of vitamin B3 or a derivative thereof.

### Summary of the invention

According to the first aspect of the present invention there is provided a sanitizer composition comprising
(i) 60 to 89 wt% of at least one C2 to C3 alcohol;
(ii) 0.1 to 10 wt% of a Vitamin B3 compound or derivatives thereof selected from niacinamide, picolinamide, isonicotinamide, cyclo-propyl niacinamide, cyclo-pentyl niacinamide or combinations thereof; and
(iii) 0.1 to 10 wt % of a non-ionic surfactant having an HLB value of at least 13 and having the general formula

   RPr (EO)mOH

   Where R is an alkyl or alkylene group of 8 to 20 carbon atoms;
   P is a phenolic group with r having a value of 0 or 1;
   And (EO) is an ethoxylate group with m having a value from 8 to 100.

According to another aspect of the present invention there is provided a method of sanitizing a topical surface of a human body, preferably the hands, comprising the step of applying a composition of the first aspect on to the desired surface.

According to yet another aspect of the present invention there is provided use of a composition of the first aspect for disinfecting a topical surface of the human body.

### Detailed description of the invention

The features and advantages of the invention will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples, embodiment and figures given in the description below are intended to clarify the invention and are not intended to limit the invention to those examples per se.

Unless otherwise specified, numerical ranges expressed in the format "from x to y" are understood to include x and y. In specifying any range of values or amounts, any particular upper value or amount can be associated with any particular lower value or amount. Except in the examples and comparative experiments, or where otherwise explicitly indicated, all numbers are to be understood as modified by the word "about". All percentages and ratios contained herein are calculated by weight unless otherwise indicated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently features specified in one section may be combined with features specified in other sections as appropriate. Any section headings are added for convenience only, and are not intended to limit the disclosure in any way. The examples are intended to illustrate the invention and are not intended to limit the invention to those examples *per se.*

A first aspect of the invention relates to a sanitizer composition comprising (i) 60 to 89 wt% of at least one C2 to C3 alcohol; (ii) 0.1 to 10 wt% of a Vitamin B3 compound or derivatives thereof; and (iii) 0.1 to 10 wt % of a non-ionic surfactant having an HLB value
of at least 13 and having the general formula

RPr (EO)mOH

Where R is an alkyl or alkylene group of 8 to 20 carbon atoms;
P is a phenolic group with r having a value of 0 or 1;
And (EO) is an ethoxylate group with m having a value from 8 to 100.

By the term "Sanitizer composition" as used herein refers to a composition that is capable of killing bacteria or of inhibiting their growth.

The term "vitamin B3 compound" as used herein refers to vitamin B3, i.e. niacin which has the structure as given below:

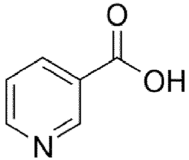

The derivatives of vitamin B3 that may be included in the composition of the invention include. niacinamide (also known as nicotinic amide or nicotinamide), picolinamide, isonicotinamide, cyclo-propyl niacinamide, cyclo-pentyl niacinamide or combinations thereof. It is especially preferred that the derivative of vitamin B3 compound for inclusion in the composition of the invention is niacinamide. Niacinamide has the structure as given below:

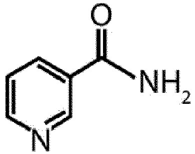

The sanitizer composition according to the invention is preferably a leave-on composition. The composition is preferably transparent.

The composition according to the invention is preferably a liquid or a thixotropic gel at 20°C. The composition according to the invention is preferably monophasic.

The composition comprises 60 to 89 wt%, preferably 60 to 80 wt%, further more preferably 60 to 75 wt% of one or more of a C₂ or C₃ alcohol. Suitable alcohols are ethanol, propanol, isopropyl alcohol and combinations thereof. Ethanol is the most preferred alcohol. The composition preferably comprises a combination of ethanol and isopropanol. When ethanol is present, it is preferably included in 60 to 80 wt% of the composition. When isopropanol is present, it is preferably included in 1 to 10 wt%, preferably 1 to 5 wt% of the composition.

In a more preferred embodiment the composition preferably comprises 0.1 to 5 wt%, more preferably 0.5 to 5 wt%, further more preferably 1 to 5 wt%, even further more preferably 2 to 4 wt% of the vitamin B3 compound or derivative thereof.

It has been observed that when the composition comprises at least 60 wt% alcohol and a vitamin B3 compound or derivative thereof is added to it, the composition suffers from becoming hazy. This problem is not very significant as the concentration of alcohol is reduced below 60 wt% and is not observed at all when it is reduced to as much as 30 wt%. Further, this problem is more significant as the concentration of vitamin B3 compound or derivative thereof is increased and is highly apparent when it is increased to more than 1 wt% of the composition. The present inventors have solved this problem by including a non-ionic surfactant of a specific class.

The non-ionic surfactant to be included has the formula:

RPr (EO)mOH

Where R is a alkyl or alkylene group of 8 to 20 carbon atoms;
P is a phenolic group with r having a value of 0 or 1;
And (EO) is an ethoxylate group with m having a value from 8 to 100.

Preferred non-ionic surfactant is selected from a fatty alcohol ethoxylate or an alkyl phenol ethoxylate.

Ethoxylates of fatty alcohol (usually available under the Brij class) have the structure: 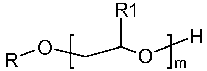 where R is a group consisting of carbon chain of 8 to 20; and R1 is alkyl or H. Suitable fatty alcohol ethoxylates for inclusion in the composition of the invention have a value of m from 20 to 100.

In the case of fatty alcohol ethoxylates, r has a value of zero i.e there is no phenolic group. When a fatty alcohol ethoxylate is included in the composition of the invention, it has an HLB value of at least 15.

HLB is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20. Typical values for various surfactants are given below:
- A value <10 : Lipid soluble (water insoluble)
- A value >10 : Water soluble
- A value from 4 to 8 indicates an anti-foaming atent
- A value from 7 to 11 indicates a W/O (water in oil) emulsifier
- A value from 12 to 16 indicates oil in water emulsion
- A value from 11 to 14 indicates a wetting agent
- A value from 12 to 15 is typical of detergents
- A value of 16 to 20 indicates a solubiliser or hydrotrope.

Suitable examples of commercially available non-ionic surfactants for use in the composition of the invention are Brij 35 (also known as Laureth-23 or HO-(C₂H₄O)₂₃C₁₂H₂₅ or polyoxyethylene lauryl ether (a C12EO23 compound) with HLB value of 16.9), Brij 700 (also known as steareth-100 or polyoxyethylene (100) stearyl ether (C18EO100) with HLB value of 18.8), or Brij 99/ Brij 98 (polyoxyethylene (20) oleyl ether (unsaturated C18EO20) with HLB value of 15.3).

When an alkyl phenol ethoxylate is the non-ionic surfactant used, the compound comprises a phenolic group and so r has a value of 1. When an alkyl phenol ethoxylate is included in the composition of the invention, m preferably has a value from 8 to 60. The compounds in this class generally are sold under the tradename of Triton. Suitable examples of these are sold under the brand names of Triton X100 (C8PhEO9.5 with HLB value of 13.4), Triton X165 (C8PhEO16 with HLB value of 15.5), Triton X305 (C8PhEO30 with HLB value of 17.3), Triton X405 (C8PhEO35 with HLB value of 17.6), or Triton X705 (C8PhEO55 with HLB value of 18.4). An especially useful compound of this class for inclusion in the composition of the invention is Triton X100. The term Ph in the above chemical formulae relates to a phenolic group.

Thus, most suitable non-ionic surfactants for inclusion in the present invention are Brij 35, Brij 98 or Triton X100 which correspond to the chemical names Laureth-23, polyoxyethylene (20) oleyl ether and a C8 phenol ethoxylate with average 9.5 ethoxylate groups, respectively. The non-ionic surfactant is included in 0.1 to 10%, preferably 0.1 to 5 %, more preferably 1 to 5 %, by weight of the composition.

The antimicrobial composition preferably comprises 1 to 50 wt % of water, more preferably 10 to 40 wt % of water, most preferably 20 to 40 wt. % of water.

The composition preferably comprises 0.01 to 1 wt % of a thickening agent, more preferably 0.05 to 0.8 wt % of a thickening agent, most preferably 0.05 to 0.5 wt % of a thickening agent.

The thickening agent in the composition according to the invention is preferably selected from homopolymers, copolymers and combinations thereof, wherein said homopolymers and copolymers comprise monomers selected from acrylic acid, acrylates, compounds comprising an acrylate group and combinations thereof. The most preferred thickening agent for use in the invention are those of the Carbomer class of compounds.

In a preferred embodiment the composition according to the invention comprises 0 to 15 wt. % of optional ingredients such as chelating agents, colorants, perfumes, skin conditioning agents, vitamins other than vitamin B3, and combinations thereof.

The composition preferably comprises skin conditioning agents and these skin conditioning agents are preferably selected from silicones, siloxanes and combinations thereof.

The composition preferably comprises vitamins other than vitamin B3 and these vitamins are preferably selected from vitamin A, vitamin E or combinations thereof. Even more preferably the composition comprises vitamin E acetate.

The method of the invention comprises sanitizing a topical surface of a human body, preferably the hands, comprising the step of applying a composition of the first aspect on to the desired surface. The method also ensures long lasting hygiene benefit by way of ensuring prolonged immunity benefit, sometimes for as long as ten hours.

The invention also relates to use of a composition of the first aspect for disinfecting a topical surface of the human body.

The method and use hereinabove are preferably for non-therapeutic or cosmetic application.

The invention will now be further described with reference to the non-limiting examples.

### Examples

### Examples A-E, 1: Effect of inclusion of non-ionics and amount of alcohol in the composition, on the haziness

### Preparation of sanitizer compositions

Three compositions as shown in Table -1 were prepared.

**Table 1**

| Example | A (wt.%) | B (wt.%) | 1 (wt.%) |
|---|---|---|---|
| Ethanol | 62 | 62 | 62 |
| Niacinamide | - | 3 | 3 |
| Brij-35 | - | - | 2 |
| Water | Up to 100% | Up to 100% | Up to 100% |

The grey scale value of the above samples were measured using the following procedure:
A drop of each sample was spread on a cover slip. They were then incubated at 24°C for 24 to 48 hours to monitor the white layer formation. The sanitizer without niacinamide (Example A) has not shown any white layer while sanitizer with 3% niacinamide (Example B) showed a uniform white layer. Further, on addition of Brij-35 (2%) breaking of white layer was observed. Then the images of the coverslip were taken and their grey scale values were measured using Image J software. The higher the grey scale value, the more hazy the product appears.

The grey scale value and the standard deviation based on three measurements is give below in Table - 2

**Table 2**

| Example | A (wt.%) | B (wt.%) | 1 (wt.%) |
|---|---|---|---|
| Grey scale value | 140 | 235 | 135 |
| Standard deviation | 4.0 | 9.5 | 8.5 |

The data in Table -2 above indicates that a hand sanitizer composition comprising niacinamide (with alcohol content above 60%, Example B) appears much hazier than one without niacinamide (Example A). The transparency is restored when a non-ionic surfactant meeting the claimed properties is included (Example 1).

Compositions with alcohol content well below 60% (at 30 wt%) were prepared as shown in Table 3 below. The grey scale values of those compositions are also given in the same table.

**Table 3**

| Example | C (wt.%) | D (wt.%) | E (wt.%) |
|---|---|---|---|
| Ethanol | 30 | 30 | 30 |
| Niacinamide | - | 3 | 3 |
| Brij-35 | - | - | 2 |
| Water | Up to 100% | Up to 100% | Up to 100% |
| Grey scale value | 135 | 138 | 136 |
| Standard deviation | 8.9 | 11.2 | 4.0 |

The data in Table 3 above indicates that the problem of haziness is not apparent when alcohol is present in an amount much below the claimed range.

### Examples F-J, 2-4: Effect of type of non-ionic surfactant and effect of HLB of the non-ionic surfactant in the composition on the haziness

The compositions as shown in Table 4 below were prepared and their grey scale value was measured, and the average of three measurements is shown in the table

**Table 4**

| Example | F (wt. %) | G (wt.%) | H (wt.%) | I (wt.%) | 2 (wt.%) | 3 (wt.%) | 4 (wt.%) | J (wt%) |
|---|---|---|---|---|---|---|---|---|
| Ethanol | 62 | 62 | 62 | 62 | 62 | 62 | 62 | 62 |
| Niacinamide | - | 3 | 3 | 3 | 3 | 3 | 3 | 3 |
| Non-ionic | - | - | Brij 97 (A) | Tween 80 (B) | Brij 35 | Brij 98 | Triton X100 | Span 80 (C) |
| Non-ionic (wt%) | - | - | 2 | 2 | 2 | 2 | 2 | 2 |
| HLB | - | - | 12.4 | 15.0 | 16.9 | 15.3 | 13.4 | 4.3 |
| Water | Up to 100% | Up to 100% | Up to 100% | Up to 100% | Up to 100% | Up to 100% | Up to 100% | Up to 100% |
| Grey scale value | 117 | 220 | 200 | 202 | 118 | 115 | 115 | Phase separation |

A - Brij 97 is a fatty alcohol ethoxylate (unsaturated C18EO10) non-ionic surfactant
B - Tween 80 is a polyoxyethylene sorbitan alkyl ester (C18:1 EO20) non -ionic surfactant
C - Span 80 is also a polyoxyethylene sorbitan alkyl ester (C18:1 EO1) non -ionic surfactant

The data in Table - 4 above indicates that the composition of the invention (Examples 2-4) exhibit reduced haziness as non-ionic surfactants as per the invention are included. When non-ionic surfactant as per the invention (but having an HLB value outside the claimed range (Example H) or when non-ionic surfactants not claimed (Examples I, J) are included, the compositions continue to exhibit high haziness.

## Claims

1. A sanitizer composition comprising
(i) 60 to 89 wt% of at least one C2 to C3 alcohol;
(ii) 0.1 to 10 wt% of a Vitamin B3 compound or derivatives thereof selected from niacinamide, picolinamide, isonicotinamide, cyclo-propyl niacinamide, cyclo-pentyl niacinamide or combinations thereof; and
(iii) 0.1 to 10 wt % of a non-ionic surfactant having an HLB value of at least 13 and **characterized by** having the general formula
RPr (EO)mOH
Where R is an alkyl or alkylene group of 8 to 20 carbon atoms;
P is a phenolic group with r having a value of 0 or 1;
And (EO) is an ethoxylate group with m having a value from 8 to 100.

2. A composition as claimed in claim 1 wherein the non-ionic surfactant is selected from a fatty alcohol ethoxylate or an alkyl phenol ethoxylate.

3. A composition as claimed in any one of the preceding claims 1 or 2 wherein when a fatty alcohol ethoxylate is included, it has an HLB value of at least 15.

4. A composition as claimed in any one of the preceding claim wherein the non-ionic surfactant is selected from selected from Laureth-23, polyoxyethylene (20) oleyl ether or a C8 phenol ethoxylate with average 9.5 ethoxylate groups.

5. A composition as claimed in any one of the preceding claims comprising 0.1 to 5 wt% of the non-ionic surfactant.

6. A composition as claimed in any one of the preceding claims wherein said C2 to C3 alcohol is ethanol, isopropanol, n-propanol or mixtures thereof, preferably ethanol.

7. A composition as claimed in any one of the preceding claims wherein said derivative of vitamin B3 compound is niacinamide.

8. A composition as claimed in any one of the preceding claims comprising 0.1 to 5% by weight of the Vitamin B3 compound or derivative thereof.

9. A composition as claimed in any one of the preceding claims comprising 1 to 50 wt%, preferably 10 to 40 wt% water.

10. A method of sanitizing a topical surface of a human body, preferably the hands, comprising the step of applying a composition as claimed in any one of the preceding claims.

11. Use of a composition as claimed in any one of the preceding claims 1 to 9 for disinfecting a topical surface of the human body.

## Patentansprüche

1. Desinfektionsmittelzusammensetzung, umfassend
(i) 60 bis 89 Gew.-% mindestens eines C2- bis C3-Alkohols;
(ii) 0,1 bis 10 Gew.-% einer Vitamin B3-Verbindung oder Derivaten davon, ausgewählt aus Niacinamid, Picolinamid, Isonicotinamid, Cyclopropylniacinamid, Cyclopentylniacinamid oder Kombinationen davon; und
(iii) 0,1 bis 10 Gew.-% eines nichtionischen Tensids mit einem HLB-Wert von mindestens 13 und **dadurch gekennzeichnet, dass** es die allgemeine Formel
Pr(EO)mOH
aufweist, wobei R eine Alkyl- oder Alkylengruppe mit 8 bis 20 Kohlenstoffatomen ist;
P eine phenolische Gruppe ist, wobei r einen Wert von 0 oder 1 hat;
und (EO) eine Ethoxylatgruppe ist, wobei m einen Wert von 8 bis 100 aufweist.

2. Zusammensetzung wie in Anspruch 1 beansprucht, wobei das nichtionische Tensid ausgewählt ist aus einem Fettalkoholethoxylat oder einem Alkylphenolethoxylat.

3. Zusammensetzung wie in irgendeinem der vorhergehenden Ansprüche 1 oder 2 beansprucht, wobei, wenn ein Fettalkoholethoxylat enthalten ist, es einen HLB-Wert von mindestens 15 aufweist.

4. Zusammensetzung wie in irgendeinem der vorhergehenden Ansprüche beansprucht, wobei das nichtionische Tensid ausgewählt ist aus Laureth-23, Polyoxyethylen (20)oleylether oder einem C8-Phenolethoxylat mit durchschnittlich 9,5 Ethoxylatgruppen.

5. Zusammensetzung wie in irgendeinem der vorhergehenden Ansprüche beansprucht, umfassend 0,1 bis 5 Gew.-% des nichtionischen Tensids.

6. Zusammensetzung wie in irgendeinem der vorhergehenden Ansprüche beansprucht, wobei der C2- bis C3-Alkohol Ethanol, Isopropanol, n-Propanol oder Mischungen davon, bevorzugt Ethanol, ist.

7. Zusammensetzung wie in irgendeinem der vorhergehenden Ansprüche beansprucht, wobei das Derivat der Vitamin B3-Verbindung Niacinamid ist.

8. Zusammensetzung wie in irgendeinem der vorhergehenden Ansprüche beansprucht, umfassend 0,1 bis 5 Gewichts-% der Vitamin B3-Verbindung oder eines Derivats davon.

9. Zusammensetzung wie in irgendeinem der vorhergehenden Ansprüche beansprucht, umfassend 1 bis 50 Gew.-%, bevorzugt 10 bis 40 Gew.-%, Wasser.

10. Verfahren zum Desinfizieren einer topischen Oberfläche eines menschlichen Körpers, bevorzugt der Hände, umfassend den Schritt des Auftragens einer Zusammensetzung wie in irgendeinem der vorhergehenden Ansprüche beansprucht.

11. Verwendung einer Zusammensetzung wie in irgendeinem der vorhergehenden Ansprüche 1 bis 9 beansprucht zum Desinfizieren einer topischen Oberfläche des menschlichen Körpers.

## Revendications

1. Composition de désinfectant comprenant
(i) de 60 à 89 % en masse d'au moins un alcool en C2 à C3 ;
(ii) de 0,1 à 10 % en masse d'un composé de vitamine B3 ou dérivés de celui-ci choisis parmi le niacinamide, picolinamide, isonicotinamide, cyclo-propylniacinamide, cyclo-pentylniacinamide ou combinaisons de ceux-ci ; et
(iii) de 0,1 à 10 % en masse d'un tensioactif non-ionique ayant une valeur HLB d'au moins 13 et **caractérisée en ce qu'**elle présente la formule générale
R Pr(EO)mOH
où R est un groupe alkyle ou alkylène de 8 à 20 atomes de carbone ;
P est un groupe phénolique avec r ayant une valeur de 0 ou 1 ;
et (EO) est un groupe éthoxylate avec m ayant une valeur de 8 à 100.

2. Composition selon la revendication 1, dans laquelle le tensioactif non-ionique est choisi parmi un éthoxylate d'alcool gras ou un éthoxylate d'alkyl phénol.

3. Composition selon l'une quelconque des revendications 1 ou 2 précédentes, dans laquelle lorsqu'un éthoxylate d'alcool gras est inclus, il présente une valeur HLB d'au moins 15.

4. Composition selon l'une quelconque des revendications précédentes, dans laquelle le tensioactif non-ionique est choisi parmi Laureth-23, oléyléther de polyoxyéthylène (20) ou un éthoxylate de phénol en C8 avec 9,5 groupes éthoxylate en moyenne.

5. Composition selon l'une quelconque des revendications précédentes comprenant de 0,1 à 5 % en masse du tensioactif non-ionique.

6. Composition selon l'une quelconque des revendications précédentes, dans laquelle ledit alcool en C2 à C3 est l'éthanol, l'isopropanol, le n-propanol ou des mélanges de ceux-ci, de préférence l'éthanol.

7. Composition selon l'une quelconque des revendications précédentes, dans laquelle ledit dérivé de composé de vitamine B3 est le niacinamide.

8. Composition selon l'une quelconque des revendications précédentes comprenant de 0,1 à 5 % en masse du composé de vitamine B3 ou dérivé de celui-ci.

9. Composition selon l'une quelconque des revendications précédentes comprenant de 1 à 50 % en masse, de préférence de 10 à 40 % en masse d'eau.

10. Procédé de désinfection d'une surface topique d'un corps humain, de préférence des mains, comprenant l'étape d'application d'une composition selon l'une quelconque des revendications précédentes.

11. Utilisation d'une composition selon l'une quelconque des revendications 1 à 9 précédentes pour la désinfection d'une surface topique du corps humain.
